# EUROPEAN PATENT SPECIFICATION

(11) **EP 0 636 421 B1**
(45) Date of publication and mention of the grant of the patent: **08.07.1998**
(21) Application number: 94111893.7
(22) Date of filing: 29.07.1994
(51) Int. Cl.: B05B 11/00, A61M 15/08, B05B 7/02

(54) **Delivery system for measured quantities of liquids especially medications**
System zur Abgabe von dosierten Mengen von Flüssigkeiten, insbesondere von Arzneimitteln
Système de distribution de quantités mesurées de liquides, et specialement de médicaments

(30) Priority: 30.07.1993 US 99386; 24.08.1993 US 111330
(43) Date of publication of application: 01.02.1995
(73) Proprietor: MESHBERG, Philip, Palm Beach, FL 33480 (US)
(72) Inventor: MESHBERG, Philip, Palm Beach, FL 33480 (US)
(74) Representative: Jack, Bruce James

(56) References cited:
- EP-A- 0 070 385
- EP-A- 0 098 939
- EP-A- 0 452 728
- EP-A- 0 509 863
- WO-A-91/13689
- WO-A-92/00812
- FR-A- 2 658 486
- US-A- 5 152 435

## Description

### Background of the Invention

### Field of the Invention

The present invention relates to a dispensing package which allows both nasal and intravenous dispensing. The invention is particularly useful for dispensing medications in accurate metered quantities into the body and allows an end user to adjust the quantity of material dispensed in each metered dose. Because the dispensing pump is mounted on the operating device, the invention can be used with conventional containers used to hold medications for intravenous injection. One type of medication which the present invention is particularly useful for dispensing is insulin, which may be administered in accurate metered doses either nasally or intravenously with the device of the present invention. Furthermore, because the pump is not mounted in the container, it may be reused -- requiring less waste than a conventional container with a pump which is thrown away with the container when the container is emptied.

### Description of the Related Art

U.S. Patent No. 3,254,803 shows a device for dispensing quantities of liquid in the form of a spray into a nasal cavity. The device of this patent uses an aerosol container mounted in a dispensing package for dispensing a metered quantity of liquid. The device of U.S. Patent No. 3,254,80203 does not, however, allow a user to select or vary the quantity of liquid delivered with each dose, since the amount delivered is dependent upon the structure of the metering valve within the aerosol container.

U.S. Patent No. 3211346 shows a device for dispensing liquid under the action of a pump, in which the pump is mounted to a non-vented container pressurized by a quantity of inert gas. This type of device, in which the material to be dispensed does not come into contact with air until it exists the spray head, is particularly useful for dispensing medications, which could degrade if placed in contact with the air used to vent a conventional piston-operated dispensing pump. The device of U.S. Patent No. 3211346 does not contain any mechanism for limiting the length of the stroke of the pump. The only metered quantity that this pump can dispense is that quantity that comes when the pump is depressed through its full stroke.

EP-A-0509863 discloses a device for dispensing liquid, contained in a container, under the action of a pump which has a hollow operating stem extending from one end of the container. The container is longitudinally slidable in a housing and the operating stem of the pump is engageable in an internal recess in a closure applied to one end of the housing. The closure has a dispensing passage extending therethrough from said recess. A cap removably securable to said one end of the container has a central stem extending axially internally. The cap can be fitted slidably on the rear end of the housing remote from the closure, with said central stem projecting through a central aperture in the end of the housing remote from the enclosure, to engage the end, remote from the pump, of said container. By pushing the cap, thus fitted to the rear end of the housing, towards the opposite end of the housing, the container body is thus pushed against the operating stem to cause the liquid to be dispensed through the operating stem and through the dispensing passage in the closure.

### Summary of the invention

It is an object of the present invention to provide a package for a material dispensing device which facilitates the relative movement of a container for the material being dispensed and an operating member projecting from the container through which the material is dispensed.

According to this invention, there is provided a device for dispensing metered quantities of material as either liquid or spray comprising:
a container holding a quantity of material to be dispensed; a pump for dispensing material from an interior of said container, said pump including a pump stem for delivering liquid from said pump to an exterior of said container; a housing including a spray passage, said pump stem engaging said spray passage and said container being mounted for reciprocal movement in said housing; and a pump stroke limiting device, comprising a washer adapted to be mounted on said pump stem between said container and said housing, such that said washer contacts a portion of said container adjacent said pump stem to thereby limit an amount of reciprocal movement of said container in said housing, the washer being replaceable with one of a plurality of other washers, each said washer being of a different thickness.

The housing can be constructed of a transparent material, so that easy visual inspection can be made of the dispersed medication, to ensure that it has not deteriorated.

It is still further an object of the invention to provide a package for material dispensing which is adapted to be manufactured by quantity production methods, is readily assembled, and is of such rugged character it will function over long periods of time with freedom from all difficulties.

### Description of the drawings

Other objects and advantages of the invention will be apparent from the specification and claims, when considered in connection with the attached sheets of drawings, illustrating one form of the invention, wherein like characters represent like parts and in which:
Figure 1 is an elevational view, in section, of a package according to a first embodiment of the invention in combination with a material dispensing device;
Figure 2 is a view, taken in the direction of the arrows 2-2 in Figure 1, of the dispensing package of Figure 1;
Figure 3 is a bottom view of the cover member of the dispensing package of Figure 1;
Figure 4 is an elevational view of the dispensing package of Figure 1, showing the material dispensing device in dotted lines, being manually operated as contemplated by the invention;
Figure 5 is a fragmentary elevational view, in section, of the nasal applicator end of the dispensing package of Figure 1;
Figure 6 is an end view of the mechanical breakup device embodied in the end of the dispensing device shown in Figure 5;
Figure 7 is a cross-sectional view of a second embodiment of the driving piston of the present invention, which includes indicia for dose and date;
Figure 8 is a bottom view of the driving piston of Figure 7;
Figure 9 is an elevational view, in section, of a package according to a second embodiment of the invention in combination with a material dispensing device;
Figure 10 is an elevational view of the dispensing package of Figure 9, showing the material dispensing device in dotted lines, being manually operated as contemplated by the invention, and
Figure 11 is a view, taken in the direction of the arrows 13-13 in Figure 9, of the dispensing package of Figure 9.

### Detailed Description of the Invention

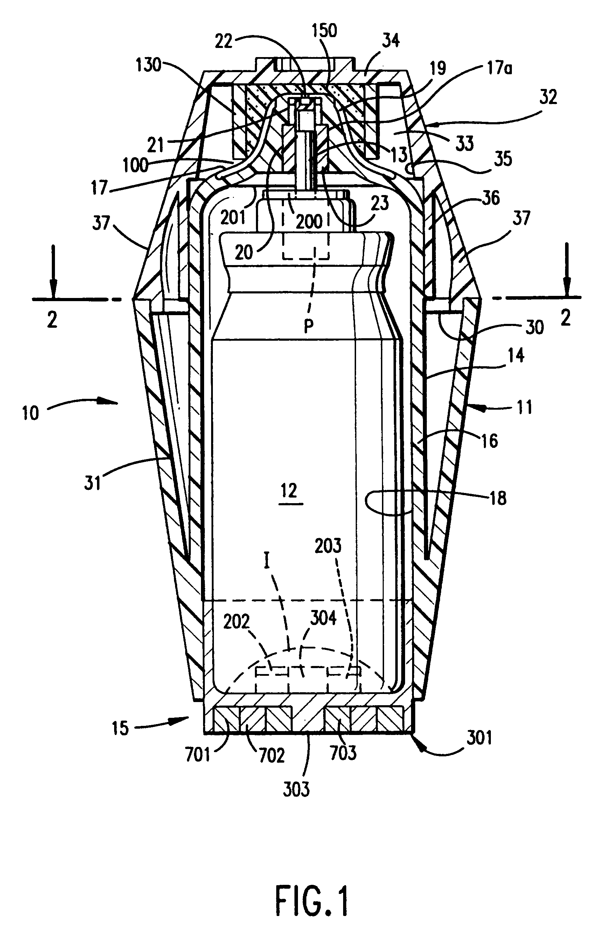

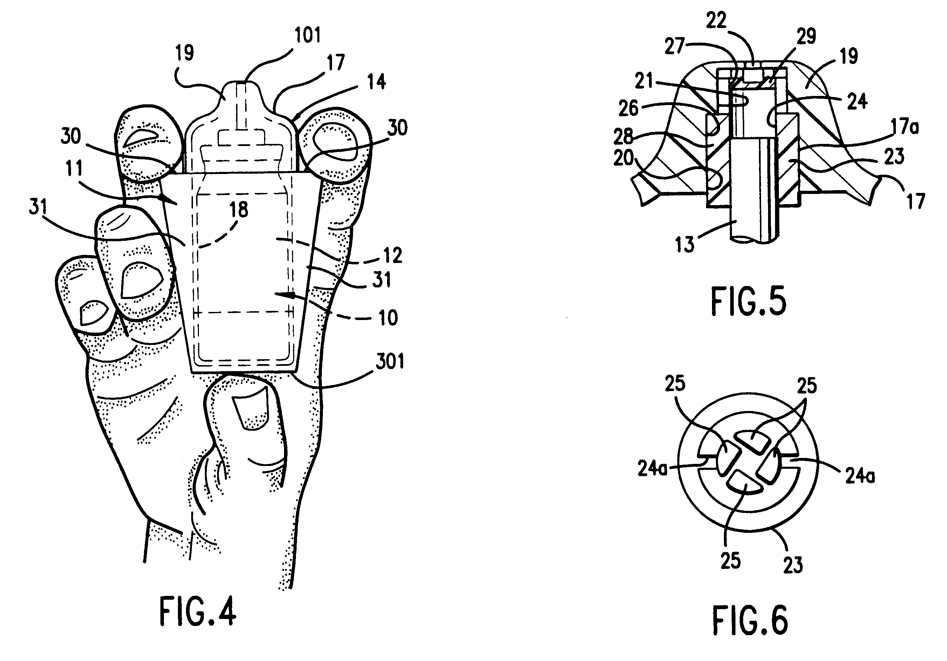

Referring now to the drawings for a more detailed description of the invention, in Figures 1 and 4 a material dispensing device 10 is shown in combination with the invention, for protecting the dispensing device and facilitating its operation.

While the packaging concepts of the present invention are applicable to a variety of dispensing devices, which include a container for the material to be dispensed and a dispensing operating member connected thereto, in the herein illustrated form of the invention the device 10 comprises a well known type of material dispensing package including a pump P and non-vented container 12 (i.e. like the pump and container shown in U.S. Patent No. 3211346) having a reciprocable operating member or pump stem 13 projecting outwardly of the container for operating the pump on being moved relative thereto, the operating member being formed with an axial passage (also not shown) through which the material is dispensed. The container **12** is preferably filled with a medication which may enter the blood stream when dispensed into the nasal cavity, for example, a medication like insulin.

It is common practice in the material dispensing art, and particularly in the field of spray pump devices, to package the dispensing device for decorative or protective reasons. In most material dispensing devices, spraying is initiated by displacement of an actuator by finger pressure. In certain devices, however, such a nasal applicators, or the like, an actuator may not be used, since the spray nozzle is inserted in the nasal passage. In such a device, another mechanism must be provided for operating the device.

The dispensing package **11**, according to the present invention, in addition to forming the conventional decorative and protective casing for the device **10**, provides the mechanism for relatively moving the material container **12** and its dispensing pump stem **13** to operate the device.

As illustrated, the package **11**, which may be molded or similarly formed from plastic or like material and is inserted into a nasal passage, includes a housing **14**, open at one end as at **15**, having a cylindrical side wall portion **16** of the substantially uniform thickness and closed end portion **17**. The side wall portion and closed end portion define within the housing a cavity **18** that substantially conforms in shape to the outline of material container **12** and is adapted to movably receive the container therein. As clearly shown in Fig. 4, the container is engageable through the opposite end **15** of the housing for applying a force thereto, as by thumb pressure or the like, for moving the container in the cavity. The thumb pressure could be applied directly to the container **12**, or as shown in Fig. 4, could be applied to a driving piston **301** slidable in the open end **15** of the housing.

The closed end portion **17** of the housing **14** is formed with recess **17*a*** which provides communication between the cavity **18** and the exterior of the housing. The recess is adapted to receive and frictionally retain the operating member **13** of the dispensing device **10** so that the passage communicates with the exterior of the housing and the pump stem **13** is held in position relative to the package **11**. While the closed end portion **17** of the housing and the recess **17*a*** therein may take many forms, depending upon the ultimate use of the device **10**, in the herein illustrated form of the invention the closed end portion is formed with a boss **19**, providing a nozzle or applicator tip for insertion into a nasal passage, and the recess includes a passage extending through the boss **19**. The boss **19** is shaped so as to limit the amount of penetration of the boss **19** into the nasal passage. The boss **19** may advantageously include one or more air grooves **100** channeled in the surface of the boss **19**. The air grooves **100** allow the user to draw in air through the nose as material is being dispensed from the package **10**, thereby assisting in the delivery of the medication in container **12** to the bloodstream of the individual user.

While it is within the concepts of the invention for the recess **17*a*** to comprise merely a bore of uniform diameter extending through the closed end portion of the housing for providing the passage for fictionally receiving the operating member of the dispensing device, in the nasal applicator illustrated, where it is desirable to diffuse or break up the body of material being dispensed the recess includes a plurality of axially aligned communicating bores **20**, **21** communicating with a bore **22** in an outer wall of the portion **17**, said bores **20**, **21** being adapted to receive and fictionally hold a mechanical breakup device **23**, the bore **24** of which receives the operating member **13** and provides passage through which the material is dispensed.

The mechanical breakup device **23**, which may take any form well known to the art, but preferably has its passage **24** interrupt by baffles, **25** or the like for providing a tortuous path **24*a*** for breaking up the material being dispensed, is locked in the recess **17*a*** to prevent its accidental displacement and discharge into the nasal passage during the dispensing operation. As illustrated most clearly in Fig. 5, the mechanical breakup device **23** is locked in the recess by forming the axially aligned bores **20**, **21** and **22** of reduced diameter relative to one another, progressing from the cavity **18** outwardly, thereby providing shoulders **26** and **27** against which shoulders **28** and **29**, formed on the breakup device, abut.

In order to dispense the material from the container **12**, by relatively moving the container and the housing carrying the dispensing operating member **13**, shoulders **30** are provided on opposite sides of the housing against which a force, such as finger pressure, may be applied -- as shown in Fig. 4 -- in a direction opposed to the force moving the container through the open end **15** of the housing. While the shoulders **30** may be provided in any known manner and take any desired form, in one form of the invention illustrated they comprise outwardly flared wall portions **31**. It will be noted that the outwardly flared wall portions **31**, which have their sides gently tapering into the surface of the housing side wall portion **16**, are of substantially the same thickness as the side wall portion to eliminate strains and the like when the dispensing package is manufactured by a molding operation or the like.

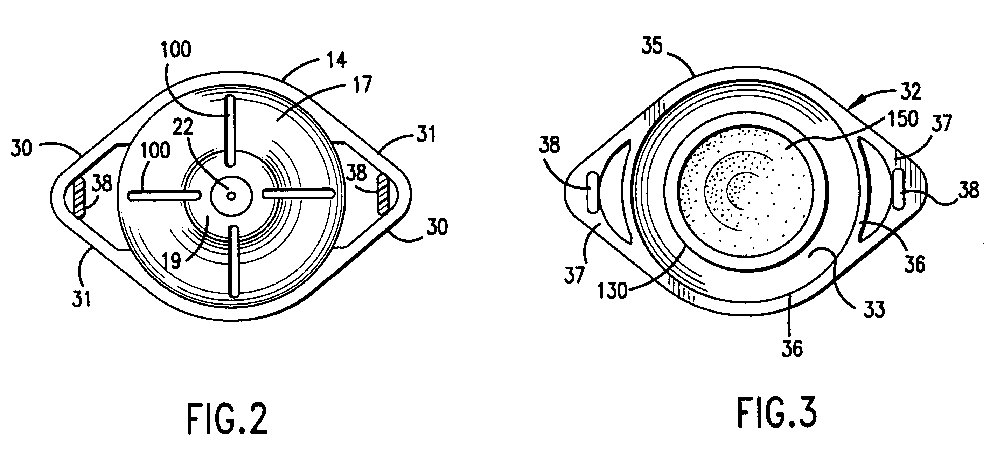

In order to complete the dispensing package **11** and protect the boss **19** when the applicator is not in use, a removable cover member **32** is provided as shown in Figs 1 and 3. The cover member, which may be formed of the same material and in a manner similar to the package housing **14** and defines a chamber **33** for receiving the closed end portion of the housing, has an end wall **34** and a side wall portion **35**, the latter including a depending annular skirt **36** for fictionally receiving a portion of the housing side wall and holding the cover member in place. In a manner similar to that described in connection with package housing, the cover member **32** is formed with opposed outwardly flared wall portions **37**, which are adapted to complement and abut the shoulders **30** of the housing for providing a symmetrical appearance to the overall package and close the pockets formed by the housing shoulders. Depending projections **38**, formed integral with outwardly flared portions **37** of the cover member, engage the shoulder **30** of the housing and extend into the pockets formed thereby for preventing the cover member form turning relative to the housing. The cover member **32** may include a device for disinfecting the boss **19** to prevent the spread and growth of microorganisms on the boss **19** between use. The disinfecting device includes a flange **130** depending downwardly from the cover member **32**, which flange holds an absorbent material **150** (e.g., a sponge-like synthetic material) which can be impregnated with a liquid disinfectant. The absorbent material **150** is shaped and positioned so that when the cover member **32** is placed on the dispensing package **11**, is surrounds and contacts the boss **19**, thereby allowing the disinfectant in the absorbent material 150 to contact the boss 19.

The device of Figure 1 includes a mechanism for limiting the stroke of the pump piston (not shown) within the container 12. A removable stop device 200, in the form of a rigid plastic washer, is placed upon the pump stem 13. The stop device 200 is therefore positioned such that it will contact the top surface of the container 12, and as the container 12 is pushed upwardly, a stop surface 201 on the closed end portion 17. When the top of the stop device 200 contacts the stop surface 201, it prevents further upward movement of the container 12, thereby limiting the stroke of the pump in the container 12, and therefore the amount of the material dispensed. The amount of material dispensed, as a function of the length of the stroke of the pump, will be controlled by the thickness of the stop device 200. In order to allow the user to vary the amount of material dispensed with a particular spray, a plurality of stop devices 202, 203 can further be provided, each of the stop devices 200, 202, 203 having a different thickness to provide a different dose size. The stop devices 200, 202, 203 can be of different colours, so that they can be easily distinguished and the various dose sizes that they provide can be determined from a quick visual inspection. The additional stop devices can be stored on a hub 304 on the driving piston 301 for easy access and storage. This hub 304 can project into an indentation I in the container 12.

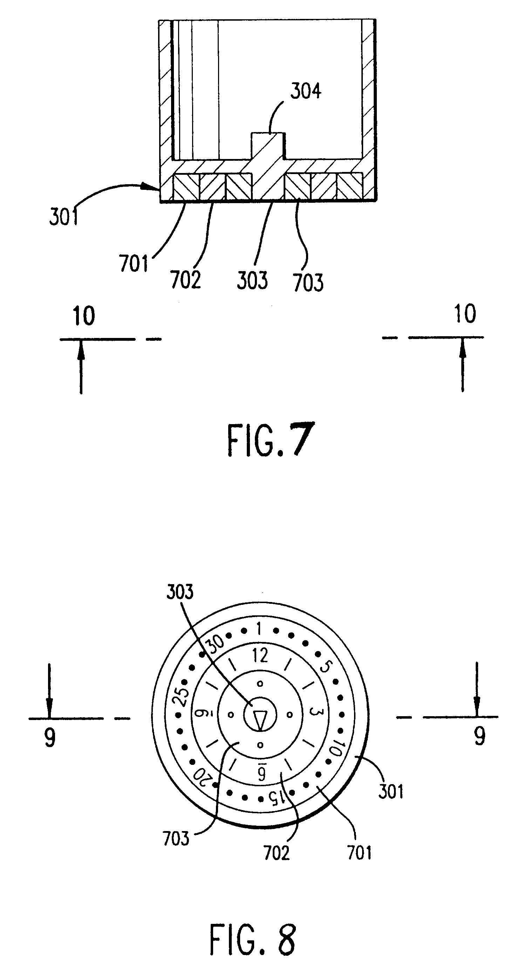

Figures 7 and 8 show an alternative embodiment of the driving piston 301. This driving piston 301 includes an indicia mechanism for indicating to the user the size, time and date of the last dose dispensed. A hub 303 is contained on the driving piston 301, the hub 303 mounting a series of indicia rings 701-703. Ring 703 can have a series of coloured dots to indicate the size of dose last dispensed, which dots can be aligned with a fixed indicia on the hub 303. Rings 702 and 701 can respectively contain rings displaying time and date of last dose, with the indicia being alignable with a fixed indicia on the hub 303. Rings 701 - 703 and hub 303 can have interlocking detents between them to allow the indicia to be snapped into a particular alignment and prevent unwanted rotation.

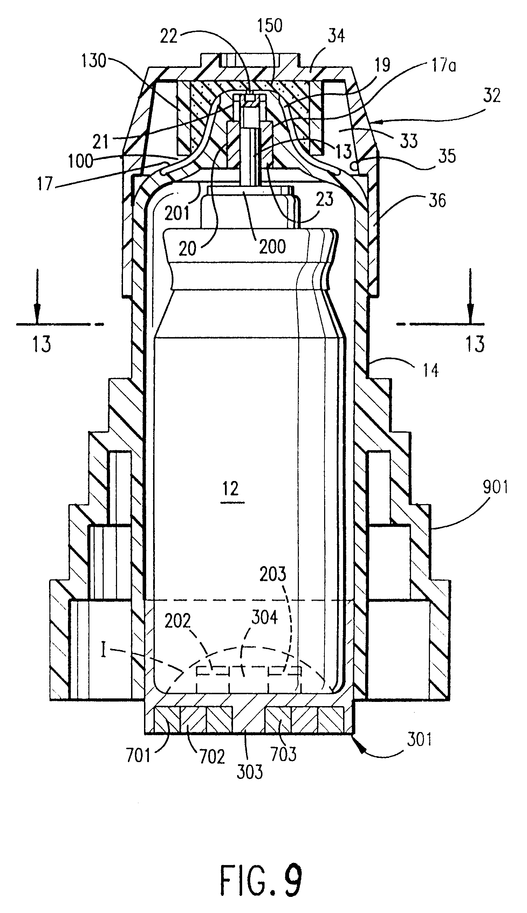

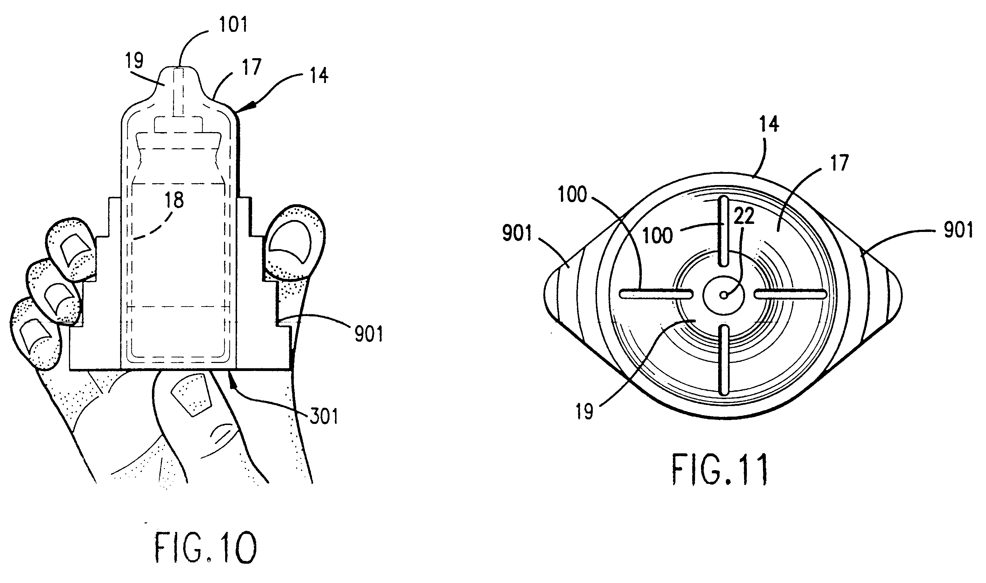

Figures 9 to 11 show a second embodiment of the present invention, with an alternate mechanism for gripping and actuating the device. The package housing 14 has depending therefrom a pair of radially projecting steps 901 extending on opposite sides of the housing 14. As can be seen particularly in Figure 10, the fingers grip these steps 901 to allow actuation of the spray pump in the container 12. The embodiment of Figures 9 to 11 is advantageous in that the fingers are spaced a distance further away from the nostril, allowing easier insertion of the device into the nostril for subsequent dispensing.

The package 11 is preferably moulded of a transparent plastic material, so that easy visual inspection of the contents of container 12 can be made. This allows the end user to tell if the medication to be dispensed is clear or cloudy, and therefore whether it is of a proper quality for safe use.

## Claims

1. A device (10) for dispensing metered quantities of material as either liquid or spray comprising;
a container (12) holding a quantity of material to be dispensed;
a pump(P) for dispensing material from an interior of said container (12), said pump(P) including a pump stem (13) for delivering liquid from said pump(P) to an exterior of said container (12);
a housing (14) including a spray passage (20), (21), said pump stem (13) engaging said spray passage (20), (21) and said container (12) being mounted for reciprocal movement in said housing (14); and characterised in that; it further comprises
a pump stroke limiting device, comprising a washer (200) adapted to be mounted on said pump stem (13) between said container (12) and said housing (14), such that said washer (200) contacts a portion of said container (12) adjacent said pump stem (13), to thereby limit an amount of reciprocal movement of said container (12) in said housing (14), the washer (200) being replaceable with one of a plurality of other washers (202,203), each said washer being of a different thickness.

2. A device according to claim 1, further comprising:
a driving piston (301) connected to said container (12) and mounted for reciprocal movement in said housing (14), whereby force applied to said driving piston (301) reciprocates said container (12) in said housing (14).

3. A device according to claim 2, wherein:
said washers (200, 202, 203) may be stored on said driving piston (301).

4. A device according to any of the proceeding claims, wherein:
said housing further comprises a spray nozzle (19), said spray nozzle (19) communicating with said pump stem spray passage (20,21).

5. A device according to claim 4, wherein:
said spray nozzle (19) further comprises at least one air passage (100) on an axially outward surface.

6. A device according to claim 4 further comprising:
a cover (32), said cover being attachable to said housing (14) to thereby cover said spray nozzle (19).

7. A device according to claim 6, wherein:
said cover (32) comprises a disinfecting device (150) for providing disinfectant to said spray nozzle (19) when said cover (32) is attached to said housing (14).

8. A device according to any one of the proceeding claims:
said housing (14) comprises at least one axially outwardly facing flange (30) to provide a surface for the application of a force opposite to a force applied to said container (12) to permit reciprocation of said container (12) in said housing (14).

9. A device according to any one of the claims 2 to 8 wherein;
said driving piston (301) comprises indicia (701-703) for indicating a time, date and quantity of a last dispensed dose.

10. A device according to claim 9, wherein:
said driving piston (301) comprises a hub (303) and said indicia (701-703) comprises a plurality of rotatable rings mounted on said hub.

11. A device according to claim 8, wherein:
said at least one axially outwardly facing flange (30) comprises a plurality of stepped flanges.

## Patentansprüche

1. Vorrichtung (10) zum Abgeben abgemessener Mengen von Material, entweder Flüssigkeit oder Spray, umfassend:
einen Behälter (12), enthaltend eine Menge abzugebendes Material;
eine Pumpe (P) zum Abgeben von Material aus dem Innenraum des Behälters (12), wobei die Pumpe (P) eine Pumpenstange (13) zum Abgeben von Flüssigkeit von der Pumpe an das Äußere des Behälters beinhaltet;
ein Gehäuse (14) mit einem Sprühdurchlaß (20), (21), wobei die Pumpenstange (13) mit dem Sprühdurchlaß in Eingriff steht und der Behälter (12) zur Hin- und Herbewegung in dem Gehäuse (14) gehalten ist; gekennzeichnet durch
eine Pumpenhubbegrenzungseinrichtung, mit einer Scheibe (200) die dazu bestimmt ist, auf der Pumpstange (13) zwischen dem Behälter (12) und dem Gehäuse (14) angebracht zu werden, so daß die Scheibe (200) mit einem Abschnitt des Behälters (12) benachbart zur Pumpenstange (13) in Kontakt steht, um dadurch die Größe der Hin- und Herbewegung des Behälters (12) in dem Gehäuse (14) zu begrenzen, wobei die Scheibe (200) durch eine Scheibe aus einer Anzahl weiterer Scheiben (202, 203) ersetzt werden kann, von deneri jede eine andere Dicke hat.

2. Vorrichtung nach Anspruch 1, weiter umfassend einen Treibkolben (301), der mit dem Behälter (12) verbunden ist und zur Hin- und Herbewegung in dem Gehäuse (14) gehalten ist, wodurch eine Kraft, die auf den Treibkolben (301) ausgeübt wird, den Behälter (12) in dem Gehäuse (14) hin- oder herbewegt.

3. Vorrichtung nach Anspruch 2, dadurch gekennzeichnet, daß die genannten Scheiben (200, 202, 203) auf dem Treibkolben (301) gelagert sein können.

4. Vorrichtung nach einem der vorangehenden Ansprüche, dadurch gekennzeichnet, daß das Gehäuse weiter eine Spraydüse (19) aufweist, die mit dem Pumpenstangenspraydurchlaß (20, 21) in Verbindung steht.

5. Vorrichtung nach Anspruch 4, dadurch gekennzeichnet, daß die Spraydüse (19) weiter zumindest einen Luftdurchlaß (100) auf einer axial außenliegenden Fläche aufweist.

6. Vorrichtung nach Anspruch 4, gekennzeichnet durch eine Abdeckung (32), die an dem Gehäuse (14) befestigbar ist, um damit die Spraydüse (19) abzudecken.

7. Vorrichtung nach Anspruch 6, dadurch gekennzeichnet, daß die Abdeckung (32) eine Desinfizierungsvorrichtung (150) beinhaltet, um einen desinfizierenden Stoff an der Spraydüse (19) bereitzustellen, wenn die Abdeckung (32) auf das Gehäuse (14) aufgesetzt wird.

8. Vorrichtung nach einem der vorangehenden Ansprüche, wobei das Gehäuse (14) zumindest einen axial nach außen weisenden Flansch (30) aufweist, um eine Fläche zum Aufbringen einer Kraft bereitzustellen, die einer auf den Behälter (12) aufgebrachten Kraft entgegengesetzt ist, um die Hin- und Herbewegung des Behälters (12) in dem Gehäuse (14) zu ermöglichen.

9. Vorrichtung nach einem der Ansprüche 2 bis 8, dadurch gekennzeichnet, daß der Treibkolben (301) Anzeigemittel (701 bis 703) zum Anzeigen von Zeit, Datum und Menge einer zuletzt abgegebenen Dosis beinhaltet.

10. Vorrichtung nach Anspruch 9, dadurch gekennzeichnet, daß der Treibkolben (301) eine Lagerung (303) aufweist, wobei die Anzeigemittel (701 bis 703) aus einer Anzahl von drehbaren, auf der Lagerung gehaltenen Ringen besteht.

11. Vorrichtung nach Anspruch 8, dadurch gekennzeichnet, daß der zumindest eine, axial nach außen weisende Flansch (30) aus einer Anzahl abgestufter Flansche besteht.

## Revendications

1. Dispositif (10) destiné à distribuer des quantités mesurées d'une matière, soit sous forme liquide, soit sous forme pulvérisée, comprenant:
un réservoir (12) contenant une certaine quantité de matière à distribuer;
une pompe (P) destinée à distribuer la mantère à partir dudit réservoir (12), ladite pompe (P) comprenant une tige (13) de pompe destinée à refouler le liquide depuis ladite pompe (P) jusqu'à l'extérieur dudit réservoir (12);
un corps (14) comprenant un passage de pulvérisation (20), (21), ladite tige (13) de pompe étant engagée dans ledit passage de pulvérisation (20),(21) et ledit réservoir (12) étant monté en vue d'un mouvement de va-et-vient dans ledit corps (14),
caractérisé en ce qu'il comprend, en outre:
un dispositif de limitation de course de pompe comprenant une rondelle (200) adaptée pour être montée sur ladite tige (13) de pompe entre ledit réservoir (12) et ledit corps (14), de telle sorte que ladite rondelle (200) vienne en contact avec une partie dudit réservoir (12) au voisinage de ladite tige (13) de pompe, de manière à limiter ainsi l'ampleur du mouvement de va-et-vient dudit réservoir (12) dans ledit corps (14), la rondelle (200) pouvant être remplacéee par une rondelle d'une pluralité d'autres rondelles (202, 203), chacune desdites rondelles ayant une épaisseur différente.

2. Dispositif selon la revendication 1, comprenant, en outre:
un piston d'entraînement (301) fixé audit réservoir (12) et monté en vue d'un mouvement de va-et-vient dans ledit corps (14), grâce à quoi la force appliquée audit piston d'entraînement (301) communique un mouvement de va-et-vient audit réservoir (12) dans ledit corps (14).

3. Dispositif selon la revendication 2, dans lequel:
lesdites rondelles (200, 202, 203) peuvent être stockées sur ledit piston d'entraînement (301).

4. Dispositif selon l'une quelconque des revendications précédentes, dans lequel:
ledit corps comprend, en outre, une buse de pulvérisation (19), ladite buse de pulvérisation (19) communiquant avec ledit passage de pulvérisation (20, 21) de la tige de pompe.

5. Dispositif selon la revendication 4, dans lequel:
ladite buse de pulvérisation (19) comprend, en outre, au moins un passage d'air (100) sur une surface axialement extérieure.

6. Dispositif selon la revendication 4, comprenant, en outre:
un couvercle (32), ledit couvercle pouvant être fixé audit corps (14) de manière à recouvrir ainsi ladite buse de pulvérisation (19).

7. Dispositif selon la revendication 6, dans lequel:
ledit couvercle (32) comprend un dispositif de désinfection (150) servant à désinfecter ladite buse de pulvérisation (19) quand ledit couvercle (32) est fixé audit corps (14).

8. Dispositif selon l'une quelconque des revendications précédentes:
ledit corps (14) comprend au moins un rebord (30) orienté axialement vers l'extérieur pour constituer une surface pour l'application d'une force opposée à une force appliquée audit réservoir (12) afin de permettre le mouvement de va-et-vient dudit réservoir (12) dans ledit corps (14).

9. Dispositif selon l'une quelconque des revendications 2 à 8, dans lequel:
ledit piston d'entraînement (301) comprend des indices (701-703) servant à indiquer un temps, une date et une quantité d'une dose distribuée en dernier.

10. Dispositif selon la revendication 9, dans lequel:
ledit piston d'entraînement (301) comprend un moyeu (303) et ledit indice (701-703) comprend une pluralité de bagues tournantes montées sur ledit moyeu.

11. Dispositif selon la revendication 8, dans lequel:
ledit rebord (30) orienté axialement vers l'extérieur et au nombre d'au moins un comprend une pluralité de rebords étagés.
